# EUROPEAN PATENT APPLICATION

(11) **EP 0 936 271 A1**
(43) Date of publication of application: **18.08.1999**
(21) Application number: 98400368.1
(22) Date of filing: 17.02.1998
(51) Int. Cl.: C12N 15/12, C07K 14/705, C12N 5/10, C07K 16/28, A61K 38/17, G01N 33/68, C12Q 1/68

(54) **Calcium-activated potassium channel beta subunit gene and protein**

(71) Applicant: SYNTHELABO, 92350 Le Plessis Robinson (FR)
(72) Inventor: Partiseti, Michel, 92160 Antony (FR)
(74) Representative: Thouret-Lemaitre, Elisabeth

(57) **Abstract**

The invention relates to hBKb3 polypeptides and polynucleotides and methods for producing such polypeptides by recombinant techniques are disclosed. The invention also relates to methods for utilizing hBKb3 polypeptides and polynucleotides in the design of protocols for the treatment of asthma, cerebral and cardiac and renal ischemias, brain and cardiac diseases, inflammation, pain, to mimic or antagonize effect of endogenous neurotransmitters and hormones, or to diagnose or treat any disorder related to abnormal expression of this protein, among others and diagnostic assays for such conditions.

SEQUENCE LISTING

(1) GENERAL INFORMATION:
5
(i) APPLICANT:
(A) NAME: SYNTHELABO
(B) STREET: 22 avenue de Galilee
(C) CITY: Le Plessis-Robinson
10 (E) COUNTRY: France
(F) POSTAL CODE (ZIP): 92350
(G) TELEPHONE: (33) 1 45 37 55 55

(ii) TITLE OF INVENTION: calcium-activated potassium channel beta
15 subunit gene and protein

(iii) NUMBER OF SEQUENCES: 2

(iv) COMPUTER READABLE FORM:
20 (A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

25 (v) CURRENT APPLICATION DATA:
APPLICATION NUMBER: FR 1

(2) INFORMATION FOR SEQ ID NO: 1:

30 (i) SEQUENCE CHARACTERISTICS:
(A) LENGTH: 1228 base pairs
(B) TYPE: nucleic acid
(C) STRANDEDNESS: double
(D) TOPOLOGY: circular
35
(ii) MOLECULE TYPE: cDNA to mRNA

(iii) HYPOTHETICAL: NO

40 (iv) ANTI-SENSE: NO

(vi) ORIGINAL SOURCE:
(A) ORGANISM: Homo sapiens

45 (ix) FEATURE:
(A) NAME/KEY: 5'UTR
(B) LOCATION:1..29

(ix) FEATURE:
50 (A) NAME/KEY: CDS
(B) LOCATION:30..659

(ix) FEATURE:
(A) NAME/KEY: 3'UTR
(B) LOCATION:660..1228

5
(xi) SEQUENCE DESCRIPTION: SEQ ID NO: 1:

GGCACGAGAG CCGCCGAGAG TGGGGGGCG ATG GCG AAG CTC CGG GTG GCT TAC 53
Met Ala Lys Leu Arg Val Ala Tyr
10 1 5

GAG TAC ACG GAA GCC GAG GAC AAG AGC ATC CGG CTC GGC TTG TTT CTC 101
Glu Tyr Thr Glu Ala Glu Asp Lys Ser Ile Arg Leu Gly Leu Phe Leu
    10                  15                  20
15
ATC ATC TCC GGC GTC GTG TCG CTC TTC ATC TTC GGC TTC TGC TGG CTG 149
Ile Ile Ser Gly Val Val Ser Leu Phe Ile Phe Gly Phe Cys Trp Leu
25                  30                  35                  40

20 AGT CCC GCG CTG CAG GAT CTG CAA GCC ACG GAG GCC AAT TGC ACG GTG 197
Ser Pro Ala Leu Gln Asp Leu Gln Ala Thr Glu Ala Asn Cys Thr Val
                45                  50                  55

CTG TCG GTG CAG CAG ATC GGC GAG GTG TTC GAG TGC ACC TTC ACC TGT 245
25 Leu Ser Val Gln Gln Ile Gly Glu Val Phe Glu Cys Thr Phe Thr Cys
60 65 70

GGC GCC GAC TGC AGG GGC ACC TCG CAG TAC CCC TGC GTC CAG GTC TAC 293
Gly Ala Asp Cys Arg Gly Thr Ser Gln Tyr Pro Cys Val Gln Val Tyr
30 75 80 85

GTG AAC AAC TCT GAG TCC AAC TCT AGG GCG CTG CTG CAC AGC GAC GAG 341
Val Asn Asn Ser Glu Ser Asn Ser Arg Ala Leu Leu His Ser Asp Glu
90 95 100
35
CAC CAG CTC CTG ACC AAC CCC AAG TGC TCC TAT ATC CCT CCC TGT TGG 389
His Gln Leu Leu Thr Asn Pro Lys Cys Ser Tyr Ile Pro Pro Cys Trp
105 110 115 120

40 AGA GAA AAT CAG AAG AAT TTG GAA AGT GTC ATG AAT TGG CAA CAG TAC 437
Arg Glu Asn Gln Lys Asn Leu Glu Ser Val Met Asn Trp Gln Gln Tyr
125 130 135

TGG AAA GAT GAG ATT GGT TCC CAG CCA TTT ACT TGC TAT TTT AAT CAA 485
45 Trp Lys Asp Glu Ile Gly Ser Gln Pro Phe Thr Cys Tyr Phe Asn Gln
140 145 150

CAT CAA AGA CCA GAT GAT GTG CTT CTG CAT CGC ACT CAT GAT GAG ATT 533
His Gln Arg Pro Asp Asp Val Leu Leu His Arg Thr His Asp Glu Ile
50 155 160 165

GTC CTC CTG CAT TGC TTC CTC TGG CCC CTG GTG ACA TTT GTG GTG GGC 581

Val Leu Leu His Cys Phe Leu Trp Pro Leu Val Thr Phe Val Val Gly
170 175 180

GTT CTC ATT GTG GTC CTG ACC ATC TGT GCC AAG AGC TTG GCG GTC AAG 629
5 Val Leu Ile Val Val Leu Thr Ile Cys Ala Lys Ser Leu Ala Val Lys
185 190 195 200

GCG GAA GCC ATG AAG AAG CGC AAG TTC TCT TAAAGGGGAA GGAGGCTTGT 679
Ala Glu Ala Met Lys Lys Arg Lys Phe Ser
10 205 210

AGAAAGCAAA GTACAGAAGC TGTACTCATC GGCACGCGTC CACCTGCGGA ACCTGTGTTT 739

CCTGGCGCAG GAGATGGACA GGGCCACGAC AGGGCTCTGA GAGGCTCATC CCTCAGTGGC 799
15
AACAGAAACA GGCACAACTG GAAGACTTGG AACCTCAAAG CTTGTATTCC ATCTGCTGTA 859

GCAATGGCTA AAGGGTCAAG ATCTTAGCTG TATGGAGTAA CTATTTCAGA AAACCCTATA 919

20 AGAAGTTCAT TTTCTTTCAA AAGTAACAGT ATATTATTTG TACAGTGTAG TATACAAACC 979

ATTATGATTT ATGCTACTTA AAAATATTAA AATAGAGTGG TCTGTGTTAT TTTCTATTTC 1039

CTTTTTTATG CTTAGAACAC CAGGGTTTAA AAAAAAAAAA AGGTGAGGAC ATCTGGGTCT 1099
25
CATTTGCTTC TGCTAGGTTA AACTTTTACT TGACAACAAG GATTCCTGCT GAAGTCTGAA 1159

CCTTACTGTG TAACCCTCAG TTTCCACTAT TAAAGAGTAT CTTTTGACGT CAAAAAAAAA 1219

30 AAAAAAAAA 1228

(2) INFORMATION FOR SEQ ID NO: 2:

35 (i) SEQUENCE CHARACTERISTICS:
(A) LENGTH: 210 amino acids
(B) TYPE: amino acid
(D) TOPOLOGY: linear

40 (ii) MOLECULE TYPE: protein
(xi) SEQUENCE DESCRIPTION: SEQ ID NO: 2:

Met Ala Lys Leu Arg Val Ala Tyr Glu Tyr Thr Glu Ala Glu Asp Lys
1               5                   10                  15
45
Ser Ile Arg Leu Gly Leu Phe Leu Ile Ile Ser Gly Val Val Ser Leu
            20                  25                  30

Phe Ile Phe Gly Phe Cys Trp Leu Ser Pro Ala Leu Gln Asp Leu Gln
50 35 40 45

Ala Thr Glu Ala Asn Cys Thr Val Leu Ser Val Gln Gln Ile Gly Glu

    50                  55                  60

Val Phe Glu Cys Thr Phe Thr Cys Gly Ala Asp Cys Arg Gly Thr Ser
65                  70                  75                  80
5
Gln Tyr Pro Cys Val Gln Val Tyr Val Asn Asn Ser Glu Ser Asn Ser
                85                  90                  95

Arg Ala Leu Leu His Ser Asp Glu His Gln Leu Leu Thr Asn Pro Lys
10 100 105 110

Cys Ser Tyr Ile Pro Pro Cys Trp Arg Glu Asn Gln Lys Asn Leu Glu
        115                 120                 125

15 Ser Val Met Asn Trp Gln Gln Tyr Trp Lys Asp Glu Ile Gly Ser Gln
130 135 140

Pro Phe Thr Cys Tyr Phe Asn Gln His Gln Arg Pro Asp Asp Val Leu
145 150 155 160
20
Leu His Arg Thr His Asp Glu Ile Val Leu Leu His Cys Phe Leu Trp
165 170 175

Pro Leu Val Thr Phe Val Val Gly Val Leu Ile Val Val Leu Thr Ile
25 180 185 190

Cys Ala Lys Ser Leu Ala Val Lys Ala Glu Ala Met Lys Lys Arg Lys
195 200 205

30 Phe Ser
210

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to β subunit of Big K potassium channel family, hereinafter referred to as human BK potassium channel β 3 subunit (hBKb3). The invention also relates to inhibiting or activating the action of such polynucleotides and polypeptides.

### BACKGROUND OF THE INVENTION

Large-conductance calcium-activated potassium channels (BK channels) represent a functional subtype of the potassium channel superfamily and are expressed in a wide range of cells such as neurons, smooth- skeletal- and cardiac-muscle cells, renal cells or also pancreatic b-cells. These channels are activated by both intracellular calcium and membrane depolarization. Their major role is probably to couple chemical signaling triggered by neurotransmitters and hormones to electrical signaling (Latorre et al., 1989, Annu. Rev. Physiol. 51, pp 385-399). For example, BK channels may regulate the amount of neurotransmitter released in neurons (Robitaille and Chralton, 1992, J. Neurosc. 12, pp 297-305) or modulate the vascular tone, either by a vasodilation acting through an increase of the open state probability or by vasoconstriction resulting from BK channel blockade. The BK channel complexes are formed of at least two subunits: an α subunit which forms the potassium-selective pore and a modulatory β subunit. This latter subunit has been shown to dramatically increase the calcium sensitivity of the channel (e. g. α+β recombinant channels are opened by a calcium elevation at more negative potentials than α recombinant channels). In addition, the β subunit has been shown to confer the sensitivity to triterpenoid compounds such as dehydrosoyasaponin I (McManus et al., 1993, Biochemistry 32, pp 6128-6133). These compounds are known to increase the open probability of the native BK channels expressed in tracheal smooth muscle cells (McManus et al., 1993). This indicates that these channels have an established, proven history as therapeutic targets. Clearly there is a need for identification and characterization of further channels which can play a role in preventing, ameliorating or correcting dysfunctions or diseases, including, but not limited to, asthma, cerebral and cardiac and renal ischemias, brain and cardiac diseases, inflammation, pain, to mimic or antagonize effect of endogenous neurotransmitters and hormones, or to diagnose or treat any disorder related to abnormal expression of this protein.

### SUMMARY OF THE INVENTION

In one aspect, the invention relates to hBKb3 polypeptides and recombinant materials and methods for their production. Another aspect of the invention relates to methods for using such hBKb3 polypeptides and polynucleotides. Such uses include the treatment of asthma, cerebral and cardiac and renal ischemias, brain and cardiac diseases, inflammation, pain, among others. In still another aspect, the invention relates to methods to identify agonists and antagonists using the materials provided by the invention, and treating conditions associated with hBKb3 imbalance with the identified compounds. Yet another aspect of the invention relates to diagnostic assays for detecting diseases associated with inappropriate hBKb3 activity or levels.

### DESCRIPTION OF THE INVENTION

### Definitions

The following definitions are provided to facilitate understanding of certain terms used frequently herein.

"hBKb3" refers, among others, to a polypeptide comprising the amino acid sequence set forth in SEQ ID NO:2, or an allelic variant thereof.

"Receptor Activity" or "Biological Activity of the Receptor" refers to the metabolic or physiologic function of said hBKb3 including similar activities or improved activities or these activities with decreased undesirable side-effects. Also included are antigenic and immunogenic activities of said hBKb3.

"hBKb3 gene" refers to a polynucleotide comprising the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of a Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxyribonucleotide, which may be unmodified RNA or DNA or modified RNA or DNA.
"Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., "Analysis for protein modifications and nonprotein cofactors"*, Meth Enzymol* (1990) 182:626-646 and Rattan et al., "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCG program package (Devereux, J., et al., *Nucleic Acids Research* (1984) 12(1):387), BLASTP, BLASTN, FASTA (Atschul, S.F. et al., *J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to hBKb3 polypeptides. The hBKb3 polypeptides include the polypeptide of SEQ ID NO:2; as well as polypeptides comprising the amino acid sequence of SEQ ID NO:2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within hBKb3 polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% are highly preferred. Preferably hBKb3 polypeptides exhibit at least one biological activity of the receptor.

The hBKb3 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the hBKb3 polypeptides are also included in the invention. A fragment is a polypeptide having an amino acid sequence that entirely is the same as part, but not all, of the amino acid sequence of the aforementioned hBKb3 polypeptides. As with hBKb3 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of hBKb3 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of hBKb3 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate receptor activity, including those with a similar activity or an improved activity, or with a decreased undesirable activity. Also included are those that are antigenic or immunogenic in an animal, especially in a human.

Preferably, all of these polypeptide fragments retain the biological activity of the receptor, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The hBKb3 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to hBKb3 polynucleotides. hBKb3 polynucleotides include isolated polynucleotides which encode the hBKb3 polypeptides and fragments, and polynucleotides closely related thereto. More specifically, hBKb3 polynucleotide of the invention include a polynucleotide comprising the nucleotide sequence set forth in SEQ ID NO:1 encoding a hBKb3 polypeptide of SEQ ID NO: 2, and polynucleotide having the particular sequence of SEQ ID NO:1. hBKb3 polynucleotides further include a polynucleotide comprising a nucleotide sequence that has at least 80% identity to a nucleotide sequence encoding the hBKb3 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under hBKb3 polynucleotides are a nucleotide sequence which has sufficient identity to a nucleotide sequence contained in SEQ ID NO:1 or contained in the cDNA insert in the plasmid deposited with the ATCC Deposit number 209476 to hybridize under conditions useable for amplification or for use as a probe or marker. Moreover, hBKb3 polynucleotide includes a nucleotide sequence having at least 80% identity to a nucleotide sequence encoding the hBKb3 polypeptide expressed by the cDNA insert deposited at the ATCC with Deposit Number 209476, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above hBKb3 polynucleotides.

A deposit containing a human hBKb3 cDNA has been deposited with the American Type Culture Collection (ATCC), 12301 Park Lawn Drive, Rockville, Maryland 20852, USA, on november 20, 1997, and assigned ATCC Deposit Number 209476. The deposited material (clone) is a DH5α strain containing the bluescript expression vector (Stratagene) that further contains the hBKb3 cDNA, referred to as "hBKb3" upon deposit. The cDNA insert is within EcoR1-Xho1 site(s) in the vector. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

hBKb3 of the invention is structurally related to other proteins of the BK channel β subunit family, as shown by the results of sequencing the cDNA of Table 1 (SEQ ID NO: 1) encoding human hBKb3. The cDNA sequence of SEQ ID NO:1 contains an open reading frame (nucleotide number 30 to 662) encoding a polypeptide of 210 amino acids of SEQ ID NO: 2. Amino acid sequence of Table 2 (SEQ ID NO: 2) has about 42% identity (using blastP version 1.4, GCG program package) in 49 amino acid residues with a quail putative potassium channel regulatory subunit (accession number: U67865; Oberst et al., 1997, Oncogene 14 pp 1109-1116). Nucleotide sequence of Table 1 (SEQ ID NO:1) has about 30% identity (using blastN version 1.4, GCG program package) in 633 nucleotide residues (corresponding to the open reading frame) with a quail potassium channel subunit (accession number: U67865). Hydrophilicity analysis of the amino acid sequence of Table 2 (SEQ ID NO:2) shows that hBKb3 protein contains two putative transmembrane segments flanking a large extracellular domain and NH₂ and COOH cytoplasmic termini. This topology is similar to the first β subunit of BK channels described (knaus et al., 1994, J. Biol. Chem. 269, pp 17274-17278).

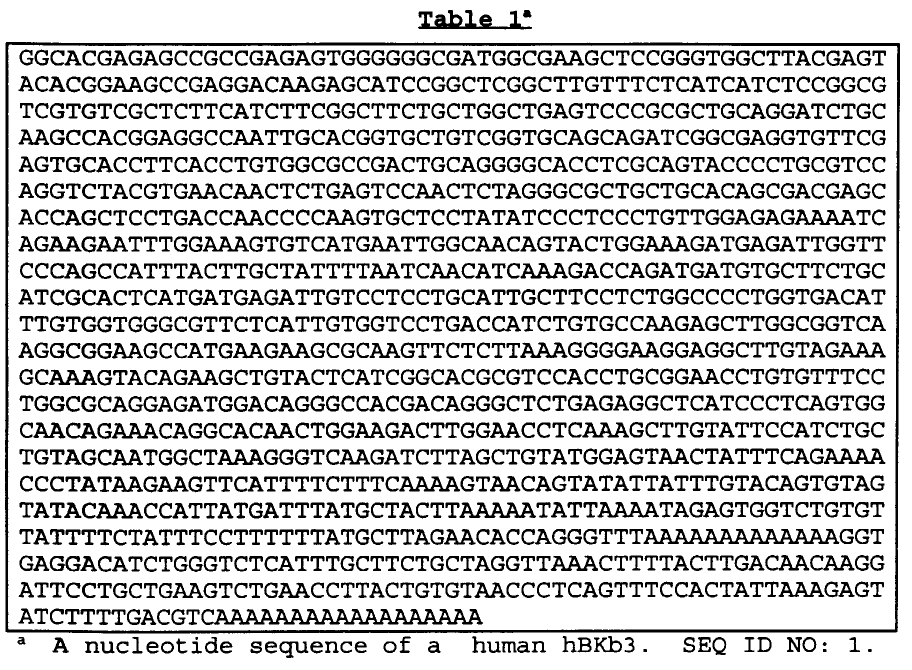

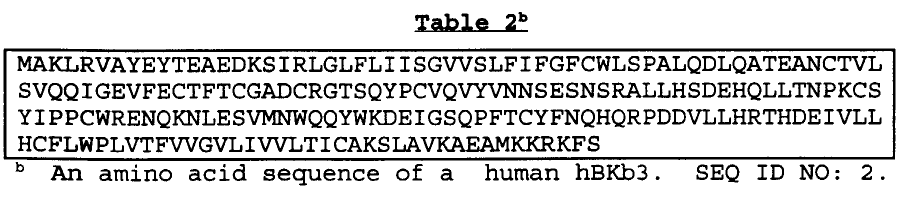

One polynucleotide of the present invention encoding hBKb3 may be obtained using standard cloning and screening, from a cDNA library derived from mRNA in cells of human spinal cord using the expressed sequence tag (EST) analysis (Adams, M.D., *et al*. *Science* (1991) 252:1651-1656; Adams, M.D. *et al*., *Nature*, (1992) 355:632-634; Adams, M.D., *et* *al*., *Nature* (1995) 377 Supp:3-174). Polynucleotides of the invention can also be obtained from natural sources such as genomic DNA libraries or can be synthesized using well known and commercially available techniques.

The nucleotide sequence encoding hBKb3 polypeptide of SEQ ID NO:2 may be identical to the polypeptide encoding sequence contained in Table 1 (nucleotide number 30 to 660 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of HBKb3 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or preproprotein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*.*, Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding hBKb3 variants comprising the amino acid sequence of hBKb3 polypeptide of Table 1 (SEQ ID NO: 2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number 209746 or a fragment thereof, may be used as hybridization probes for cDNA and genomic DNA, to isolate full-length cDNAs and genomic clones encoding hBKb3 and to isolate cDNA and genomic clones of other genes that have a high sequence similarity to the hBKb3 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding hBKb3 polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E*. *coli*, *Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook et al., *MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the hBKb3 polypeptide is to be expressed for use in screening assays, generally, it is preferred that the polypeptide be produced at the surface of the cell. In this event, the cells may be harvested prior to use in the screening assay. If hBKb3 polypeptide is secreted into the medium, the medium can be recovered in order to recover and purify the polypeptide; if produced intracellularly, the cells must first be lysed before the polypeptide is recovered.

hBKb3 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of hBKb3 polynucleotides for use as diagnostic reagents. Detection of a mutated form of hBKb3 gene associated with a dysfunction will provide a diagnostic tool that can add to or define a diagnosis of a disease or susceptibility to a disease which results from under-expression, over-expression or altered expression of hBKb3. Individuals carrying mutations in the hBKb3 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled hBKb3 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al*., *Science* (1985) 230:1242. Sequence changes at specific locations may also be revealed by nuclease protection assays, such as RNase and S1 protection or the chemical cleavage method. See Cotton *et al*., *Proc Natl Acad Sci USA* (1985) 85: 4397-4401. In another embodiment, an array of oligonucleotides probes comprising hBKb3 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability (see for example: M.Chee et al., Science, Vol 274, pp 610-613 (1996)).

The diagnostic assays offer a process for diagnosing or determining a susceptibility to asthma, cerebral and cardiac and renal ischemias, brain and cardiac diseases, inflammation and pain through detection of mutation in the hBKb3 gene by the methods described.

In addition, asthma, cerebral and cardiac and renal ischemias, brain and cardiac diseases, inflammation and pain, can be diagnosed by methods comprising determining from a sample derived from a subject an abnormally decreased or increased level of hBKb3 polypeptide or hBKb3 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an hBKb3, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridise with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the hBKb3 polypeptides. The term "immunospecific" means that the antibodies have substantial greater affinity for the polypeptides of the invention than their affinity for other related polypeptides in the prior art.

Antibodies generated against the hBKb3 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al*., *Immunology Today* (1983) 4:72) and the EBV-hybridoma technique (Cole *et al*., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against hBKb3 polypeptides may also be employed to treat asthma, cerebral and cardiac and renal ischemias, brain and cardiac diseases, inflammation, pain, to mimic or antagonize effect of endogenous neurotransmitters and hormones, among others.

### Vaccines

Another aspect of the invention relates to a method for inducing an immunological response in a mammal which comprises inoculating the mammal with hBKb3 polypeptide, or a fragment thereof, adequate to produce antibody and/or T cell immune response to protect said animal from asthma, cerebral and cardiac and renal ischemias, brain and cardiac diseases, inflammation and pain, among others. Yet another aspect of the invention relates to a method of inducing immunological response in a mammal which comprises, delivering hBKb3 polypeptide via a vector directing expression of hBKb3 polynucleotide in vivo in order to induce such an immunological response to produce antibody to protect said animal from diseases.

Further aspect of the invention relates to an immunological/vaccine formulation (composition) which, when introduced into a mammalian host, induces an immunological response in that mammal to a hBKb3 polypeptide wherein the composition comprises a hBKb3 polypeptide or hBKb3 gene. The vaccine formulation may further comprise a suitable carrier. Since hBKb3 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The hBKb3 polypeptide of the present invention may be employed in a screening process for compounds which bind the receptor and which activate (agonists) or inhibit activation of (antagonists) the receptor polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics (See Coligan *et al*., *Current Protocols in Immunology* 1(2) :Chapter 5 (1991))

hBKb3 polypeptides are responsible for many biological functions, including many pathologies. Accordingly, it is desirous to find compounds and drugs which stimulate hBKb3 on the one hand and which can inhibit the function of hBKb3 on the other hand. In general, agonists are employed for therapeutic and prophylactic purposes for such conditions as asthma, cerebral and cardiac ischemias, brain and cardiac diseases, inflammation and pain. Antagonists may be employed for a variety of therapeutic and prophylactic purposes for such conditions as renal ischemia.

In general, such screening procedures involve producing appropriate cells which express the receptor polypeptide of the present invention on the surface thereof. Such cells include cells from mammals, yeast, *Drosophila* or *E*. *coli*. Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the channel, using detection systems appropriate to the cells bearing the channel at their surfaces. Inhibitors of activation are generally assayed in the presence of a previously identified agonist and the effect on activation by the agonist by the presence of the candidate compound is observed. Standard methods for conducting such screening assays are well understood in the art.

In order to perform a functionnal test, the hBKb3 subunit can be associated to the α subunit of BK channels (accession number: U11058). Thus, The recording of recombinant (α subunit + hBKb3) channel activity may be carried out either by membrane voltage analysis of transfected cells or microinjected xenope oocytes, directly (patch-clamp for example) or indirectly (fluorescent probes sensitive to membrane potential for example). Further, these assays may test whether the candidate compound results in a signal generated by activation of the channel, using detection systems appropriate to the cells bearing the channel at their surfaces. However, The assays may simply test binding of a candidate compound wherein adherence to the cells bearing the hBKb3 protein is detected by means of a label directly or indirectly associated with the candidate compound or in an assay involving competition with a labeled competitor.

Examples of potential hBKb3 antagonists include antibodies or, in some cases, oligonucleotides or proteins which are closely related to the ligand of the hBKb3, e.g., a fragment of the ligand, or small molecules which bind to the receptor but do not elicit a response, so that the activity of the receptor is prevented.

### Prophylactic and Therapeutic Methods

This invention provides methods of treating an abnormal condition related to both an excess of and insufficient amounts of hBKb3 activity.

If the activity of hBKb3 is in excess, several approaches are available. One approach comprises administering to a subject an inhibitor compound (antagonist) as hereinabove described along with a pharmaceutically acceptable carrier in an amount effective to inhibit activation by blocking binding of ligands to the hBKb3, or by inhibiting a second signal, and thereby alleviating the abnormal condition.

In another approach, soluble forms of hBKb3 polypeptides still capable of binding the ligand in competition with endogenous hBKb3 may be administered. Typical embodiments of such competitors comprise fragments of the hBKb3 polypeptide.

In still another approach, expression of the gene encoding endogenous hBKb3 can be inhibited using expression blocking techniques. Known such techniques involve the use of antisense sequences, either internally generated or separately administered. See, for example, *O'Connor*, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al*., *Nucleic Acids Res* (1979) 6:3073; *Cooney et al*., *Science* (1988) 241:456; Dervan *et al*., *Science* (1991) 251:1360. These oligomers can be administered *per se* or the relevant oligomers can be expressed *in vivo*.

For treating abnormal conditions related to an under-expression of hBKb3 and its activity, several approaches are also available. One approach comprises administering to a subject a therapeutically effective amount of a compound which activates hBKb3, i.e., an agonist as described above, in combination with a pharmaceutically acceptable carrier, to thereby alleviate the abnormal condition. Alternatively, gene therapy may be employed to effect the endogenous production of hBKb3 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20, *Gene Therapy and other Molecular Genetic-based Therapeutic Approaches*, (and references cited therein) in Human Molecular Genetics, T Strachan and A P Read, BIOS Scientific Publishers Ltd (1996).

### Formulation and Administration

Peptides, such as the soluble form of hBKb3 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

The sequence of the hBKb3 subunit was first identified by searching a database containing approximately 1 million human ESTs, which was generated using high throughput automated DNA sequence analysis of randomly selected human cDNA clones (Adams, M.D. *et al*., *Nature 377*:3-174 (1995); Adams, M.D. *et al*., *Nature 355*:632-634 (1992); and Adams, M.D. *et al*., *Science 252*:1651-1656 (1991)). Sequence homology comparisons of each EST were performed against the GenBank database using the blastn and tblastn algorithms (Altschul, S.F. *et al*., *J*. *Mol*. *Biol*. *215*:403-410 (1990)). A specific homology search using the known human BK potassium channel beta subunit amino acid sequence (accession number: S62905, Meera et al., 1996, FEBS Letters 382 pp 84-88) against this human EST database revealed one EST, from a spinal cord cDNA library, with approximatively 21% identity and 50% similarity on a 187 amino acids overlap. The sequence comparison suggested that it contained the complete open reading frame of a new protein. Sequence of the gene was confirmed by double strand DNA sequencing using the TaqFs (Perkin Elmer) and the gene was shown to be completely new by a blast search against Genbank release 103. The entire hBKb3 coding region containing the EcoRI-XhoI fragments was inserted into the expression vector pBlueScript (Stratagene).

### Example 2

### Cloning and Expression of hBKb3 in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3 (Invitrogen). Mammalian host cells that could be used include, human HEK 293, H9 and Jurkat cells, mouse NIH3T3 and C127 cells, Cos 1, Cos 7 and mouse L cells and Chinese hamster ovary (CHO) cells (commercially available from ATCC).

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., *Biochem*. *J*. *227*:277-279 (1991); Bebbington *et al*., *Bio*/*Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pCMVsport3.0 contains the strong promoter (CMV) of the Cytomegalovirus. Multiple cloning sites, e.g., with the restriction enzyme cleavage sites EcoRI, XhoI, facilitate the cloning of the gene of interest.

### Example3

### Tissue distribution of hBKb3 mRNA expression

Northern blot analysis can be carried out to examine hBKb3 gene expression in human tissues, using methods described by, among others, Sambrook *et al*., cited above. A cDNA probe containing the entire nucleotide sequence of the hBKb3 protein can be labeled with ³²P using the Rediprime^{™} DNA labeling system (Amersham Life Science, Arlington, IL), according to manufacturer's instructions. After labeling, the probe can be purified using a CHROMA SPIN- 100^{™} column (Clontech Laboratories, Inc.), according to manufacturer's protocol number PT1200-1. The purified labeled probe was then used to examine various human tissues for hBKb3 mRNA.

Multiple Tissue Northern (MTN) blots containing various human tissues can be obtained from Clontech and examined with the labeled probe using ExpressHyb^{™} hybridization solution (Clontech) according to manufacturer's protocol number PT1190-1. Following hybridization and washing, the blots can be mounted and exposed to film at -70°C overnight, and films developed according to standard procedures.

## Claims

1. An isolated polynucleotide comprising a nucleotide sequence that has at least 80% identity to a nucleotide sequence encoding the hBKb3 polypeptide of SEQ ID NO:2 over its entire length; or a nucleotide sequence complementary to said nucleotide sequence.

2. The polynucleotide of claim 1 which is DNA or RNA.

3. A polynucleotide according to one of claims 1 and 2 wherein said nucleotide sequence is at least 80% identical to that contained in SEQ ID NO:1.

4. A polynucleotide according to one of claims 1 and 3 wherein said nucleotide sequence comprises the hBKb3 polypeptide encoding sequence contained in SEQ ID NO:1.

5. A polynucleotide according to one of claims 1 and 4 which is polynucleotide of SEQ ID NO: 1.

6. An isolated hBKb3 polynucleotide comprising a nucleotide sequence selected from teh group consisting of :
(a) a nucleotide sequence having at least 80% identity to a nucleotide sequence encoding the hBKb3 polypeptide expressd by the cDNA insert deposited at the ATCC with Deopisu Number 209476 ; and
(b) a nucleotide sequence complementary to the nucleotide sequence of (a).

7. A DNA or RNA molecule comprising an expression system, wherein said expression system is capable of producing a hBKb3 polypeptide comprising an amino acid sequence, which has at least 80% identity with the polypeptide of SEQ ID NO:2 when said expression system is present in a compatible host cell.

8. A host cell comprising the expression system of claim 7.

9. A process for producing a hBKb3 polypeptide comprising culturing a host of claim 8 under conditions sufficient for the production of said polypeptide and recovering the polypeptide from the culture.

10. A process for producing a cell which produces a hBKb3 polypeptide thereof comprising transforming or transfecting a host cell with the expression system of claim 7 such that the host cell, under appropriate culture conditions, produces a hBKb3 polypeptide.

11. A hBKb3 polypeptide comprising an amino acid sequence which is at least 80% identical to the amino acid sequence of SEQ ID NO:2 over its entire length.

12. The polypeptide of claim 11 which comprises the amino acid sequence of SEQ ID NO:2.

13. An antibody immunospecific for the hBKb3 polypeptide of claim 11.

14. Use of (a) a therapeutically effective amount of an agonist of hBKb3 polypeptide of claim 11 and/or (b) a polynucleotide according to one of claims 1 to 6 in a form so as to effect production of said hBKb3 polypeptide activity *in vivo*, for the manufacture of a medicament for the treatment of a subject in need of enhanced activity or expression of hBKb3 polypeptide.

15. Use of (a) a therapeutically effective amount of an antagonist of hBKb3 polypeptide of claim 11 and/or (b) a nucleic acid molecule that inhibits the expression of the nucleotide sequence encoding said hBKb3 polypeptide and/or (c) a therapeutically effective amount of a polypeptide that competes with said hBKb3 polypeptide, for the manufacture of a medicament for the treatment of a subject having need to inhibit activity or expression of hBKb3 polypeptide.

16. A process for diagnosing a disease or a susceptibility to a disease in a subject related to expression or activity of hBKb3 polypeptide of claim 11 in a subject comprising:
(a) determining the presence or absence of a mutation in the nucleotide sequence encoding said hBKb3 polypeptide in the genome of said subject; and/or
(b) analyzing for the presence or amount of the hBKb3 polypeptide expression in a sample derived from said subject.

17. A method for identifying agonists to hBKb3 polypeptide of claim 11 comprising:
(a) contacting cells produced by claim 10 with a candidate compound; and
(b) determining whether the candidate compound effects a signal generated by activation of the hBKb3 polypeptide.

18. An agonist identified by the method of claim 17.

19. The method for identifying antagonists to hBKb3 polypeptide of claim 11 comprising:
(a) contacting said cell produced by claim 10 with an agonist; and
(b) determining whether the signal generated by said agonist is diminished in the presence of a candidate compound.

20. An antagonist identified by the method of claim 19.
